# EUROPEAN PATENT APPLICATION

(11) **EP 2 604 229 A1**
(43) Date of publication of application: **19.06.2013**
(21) Application number: 11193430.3
(22) Date of filing: 14.12.2011
(51) Int. Cl.: A61F 2/90

(54) **Support structure for an endoprothesis, an endoprothesis and a manufacturing method therefore**

(71) Applicant: Occlutech Holding AG, 8201 Schaffhausen (CH)
(72) Inventor: Joergensen, Ib Erling, 72401 Haigerloch (DE); Nielsen, Stevan, 72108 Rottenburg Am Neckar (DE); Seibold, Gerd, 72119 Ammerbuch (DE); Quint, Bodo, 72108 Rottenburg (DE)
(74) Representative: KIPA AB

(57) **Abstract**

The disclosure is related to an endoprothesis, such as a stent. The disclosure also relates to a support structure for an endoprothesis and methods of delivering such an endoprothesis as well as a method of manufacturing a support structure. Furthermore, the disclosure provides an improved positioning of an endoprothesis and overcomes the folding issues and thus improves the flow through a body vessel. One embodiment provides a tubular support structure for avoiding folding of an endoprothesis (1), such as a stent or a valve, comprising one or more first circumferential zigzag patterns, at least two second circumferential zigzag patterns and wherein the first and second circumferential zigzag patterns are overlapping.

## Description

### Field of the Invention

This disclosure pertains in general to the field of medical implants or endoprotheses. More particularly the disclosure relates to a support structure for an endoprothesis, an endoprothesis and a method of manufacturing such a support structure.

### Background of the Invention

In general it is an issue to insert short stents with a large diameter, into a body vessel, such as a blood vessel.

Furthermore there are drawbacks associated with the positioning of an implant or an endoprothesis, such as a stent. The positioning of tubular implants is especially critical when the length of the implant is in the same range as the diameter of the implant after implantation. These issues are most common when a large tubular implant has to be implanted, e.g. aorta stents, or a heart valve, such as a transcatheter heart valve.

There are also difficulties when a tubular implant has a significantly larger diameter than the body vessel, in which it is expanded, since there is a risk that the implant is not expanding fully into a circular tubular shape. Instead it is often seen that the circumference of the implant folds inwards, i.e. there is a folding issue. This makes a restriction inside the vessel, which disturbs the natural blood flow through the vessel. This issue could occur when an implant is placed in a body vessel, having stenosis, abnormal narrowing, without pre- or post dilatation. If the implant is intended to migrate through the vessel, the implantation diameter is also significantly lower than the maximal expansion diameter of the implant.

The systems for delivering the endoprothesis can be divided into two types: self-expanding systems and external force expandable systems. For self-expanding systems, fixation systems have been developed. For external force expandable systems, long balloons are used. These solutions have major disadvantages.

For the self-expanding systems there are issues at stent release, e.g. tilting and jumping.

One prior art document, W02004/078065 A2, solves this issue with a fixation system. However this prior art document disclose a complex, inflexible and bulky delivery system.

There are also issues associated with external force expandable systems, such as balloon expandable systems.

One issue associated with these systems is sliding of the endoprothesis distally or proximally during expansion.

Another issue is that the endoprothesis has to be rigid longitudinally, since it is affected by a longitudinal force during expansion, the so called dog bone effect.

The prior art attempts to solve these issues by the use of long balloons and stiff implants/endoprotheses. However, there are some disadvantages associated with these prior art solutions, such as overhang from the long balloons and unnecessary expansion of healthy vessel area.

In addition the stiffness of the endoprothesis or implants used in prior art make them inflexible.

Thus, there is a need for improved positioning of an endoprothesis in a body vessel.

There is also a need for controlling the cross-sectional shape of an endoprothesis in its expanded shape.

In addition, it would be advantageous to provide an endoprothesis, which has an optimized ratio between the maximum diameter of the endoprothesis in expanded state and the body vessel diameter.

It would also be advantageous to provide a less complex and more flexible delivery system and to overcome disadvantages associated with the prior art.

### Summary of the Invention

Accordingly, embodiments of the present disclosure preferably seek to mitigate, alleviate or eliminate one or more deficiencies, disadvantages or issues in the art, such as the above-identified, singly or in any combination by providing a support structure for an endoprothesis, an endoprothesis and methods of delivering such an endoprothesis, as well as a method of manufacturing such a support structure, according to the appended patent claims.

An object is to overcome the disadvantage with the prior art that there may be a folding issue, i.e. the endoprothesis may fold inwards, which makes a restriction inside the vessel, and thus disturbs the natural blood flow through the vessel.

Another object is to overcome the disadvantages with the positioning of an endoprothesis in the prior art.

The present disclosure overcomes at least some of these disadvantages, amongst others, by providing a tubular support structure for avoiding folding of an endoprothesis and at least one element, such as an anchor or fixation part, attached to the endoprothesis via at least one interconnecting element, such as a hinge.

Embodiments of the present disclosure may be well suited for different types of stents, such as aorta stents, coronary stents, urethal stents, prostatic stents, sphinchter stents, esophageal stents, biliary stents, bare-metal stents, etc. and valves, such as heart valves, or even occluders for the selective occlusion of a vessel, lumen, channel, hole, cavity, or the like.

According to one aspect of the disclosure, a tubular support structure for avoiding folding of an endoprothesis, such as a stent or a valve, which comprises one or more first circumferential zigzag patterns and at least two second circumferential zigzag patterns, and wherein the first and second circumferential zigzag patterns are overlapping, is provided. The first and second circumferential zigzag patterns could also be referred to as first and second axial zigzag patterns.

According to another aspect of the disclosure, an endoprothesis, such as a stent, comprising the support structure is provided.

According to yet another aspect of the disclosure, a method of delivering an endoprothesis, preferable a self-expanding endoprothesis, through a body opening to a target site in a body is provided. The method comprises positioning of an endoprothesis inside an outer catheter. Further, insertion of the outer catheter is performed together with the endoprothesis into the body. Moreover, the method comprises positioning of a distal end of the outer catheter and the endoprothesis at the target site inside the body opening. Then the endoprothesis is delivered to the target site within the body through the outer catheter and the outer catheter is removed from the body.

According to yet another aspect of the disclosure, a method of delivering a method of delivering an endoprothesis, preferable an external force expandable endoprothesis, through a body opening to a target site in a body is provided. The method comprises positioning of an endoprothesis outside a delivery element, such as a balloon, the delivery element being short, i.e. not being substantially longer than the endoprothesis. The method also comprises insertion of the delivery element together with the endoprothesis into the body and positioning of the delivery element and the endoprothesis at the target site. Furthermore, the method comprises expansion of the delivery element, preferably by inflation, thereby expanding the endoprothesis and further expansion of the endoprothesis into its released shape. Moreover, the method comprises contraction of the delivery element, preferably by deflation, and removing of the delivery element.

According to a further aspect of the disclosure, a method of manufacturing a support structure is provided. The method comprises: providing a solid tube, the tube selected from a group comprising stainless steel, titanium, nickel titanium, tantalum, magnesium, cobalt, chromium, a magnesium alloy or a resorbable polymer. Moreover, optionally the method comprises grinding an outer surface of the tube. Thereafter heat treating annealing the tube is performed. Further, the method comprises cutting the tube to predetermined lengths for formation of the endoprothesis. The method may optionally also comprise laser cutting of the outer surface of the tube to a predetermined wall surface pattern, including the one or more first circumferential zigzag patterns and the at least two second circumferential zigzag patterns in a repeated pattern along the longitudinal axis of the endoprothesis. Alternatively, the method may comprise forming from a first part of the tube, the one or more first circumferential zigzag patterns, and forming from at least a second part of the tube or at least a part from another tube, the at least two second circumferential zigzag patterns and joining the parts together with a technique, such as welding. Thereafter sterilizing of the endoprothesis is performed. The method may comprise crimping of the endoprothesis onto a balloon for direct placement of the endoprothesis if the endoprothesis is an external force expandable endoprothesis. Alternatively, if the endoprothesis is a self-expanding endoprothesis, the method may comprise putting the endoprothesis into an outer catheter. The method comprises packaging of the endoprothesis to preserve the sterilized condition of the endoprothesis until use.

Further embodiments of the disclosure are defined in the dependent claims, wherein features for the subsequent aspects of the disclosure are as for the above given aspects mutatis mutandis.

Some embodiments of the disclosure provide for an advantageous low circumferential force, without sacrificing strength and overcoming the folding issues and thus improving the flow through the vessel.

Some embodiments of the disclosure also provide for optimization of the ratio between the maximum diameter of the endoprothesis in expanded state and the vessel diameter.

Some embodiments of the disclosure also provide for additional wall coverage as well as an elastic force perpendicular to the surface of the endoprothesis.

Some embodiments of the disclosure also simplify the method of manufacturing the structure.

Some embodiments of the disclosure also provide for improving the positioning of the endoprothesis.

Some embodiments of the disclosure also provide for enabling further fixation parts or the division of a fixation part into different sections, such as at least one distal element, such as a fixation section, and/or at least one proximal element, such as a fixation section.

Some embodiments of the disclosure also provide for avoiding undesired cross-sectional shapes and aiding in overcoming the folding issues and thus further improving the flow through the vessel.

Some embodiments of the disclosure also provide for a safer delivery mechanism and/or simplifying the delivery and/or enabling the delivery of further objects with the same delivery mechanism.

Some embodiments of the disclosure also provide for avoiding issues with prior art such as long balloon overhang and unnecessary expansion of healthy vessel area.

In one embodiment of the disclosure one or more first circumferential zigzag patterns is shaped of a substantially straight plurality of first struts and a second circumferential zigzag pattern is shaped of a substantially straight plurality of second struts.

In other embodiments the tubular support structure may be implemented in a material, such as a metal, e.g. magnesium, cobalt, chromium, titanium, tantalum, or stainless steel, such as stainless steel 316L, an alloy, e.g. nitinol or magnesium alloys, or a resorbable polymer.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the disclosure are capable of will be apparent and elucidated from the following description of embodiments of the present disclosure, reference being made to the accompanying drawings, in which
Fig. 1 is a lateral view of an endoprothesis;
Fig. 2 is another lateral view of an endoprothesis; showing the structure of the endoprothesis;
Fig. 3 is a lateral view of a delivery system for delivering an endoprothesis;
Fig. 4a is a lateral view of an endoprothesis inside an outer catheter for delivery;
Fig. 4b is a lateral view of an endoprothesis partly inside an outer catheter for delivery and partly released into its released shape;
Fig. 4c is a front view of an endoprothesis partly released into its released shape;
Fig. 5 is a lateral view of an endoprothesis after being completely released from the outer catheter;
Fig. 6a is a lateral view of an endoprothesis mounted on a delivery element, such as a balloon, when the balloon is in its deflated shape; and
Fig. 6b is a lateral view of an endoprothesis mounted on a delivery element, such as a balloon, when the balloon is in its expanded shape.

### Description of embodiments

Specific embodiments of the disclosure now will be described with reference to the accompanying drawings. This disclosure may, however, be embodied in many different forms and should not be construed as limited to the embodiments set forth herein; rather, these embodiments are provided so that this disclosure will be thorough and complete, and will fully convey the scope of the disclosure to those skilled in the art. The terminology used in the detailed description of the embodiments illustrated in the accompanying drawings is not intended to be limiting of the disclosure. In the drawings, like numbers refer to like elements.

The following description focuses on an embodiment of the present disclosure applicable to a tubular support structure and in particular to an endoprothesis comprising such a tubular support structure. However, it will be appreciated that the disclosure is not limited to this application but may be applied to many other applications.

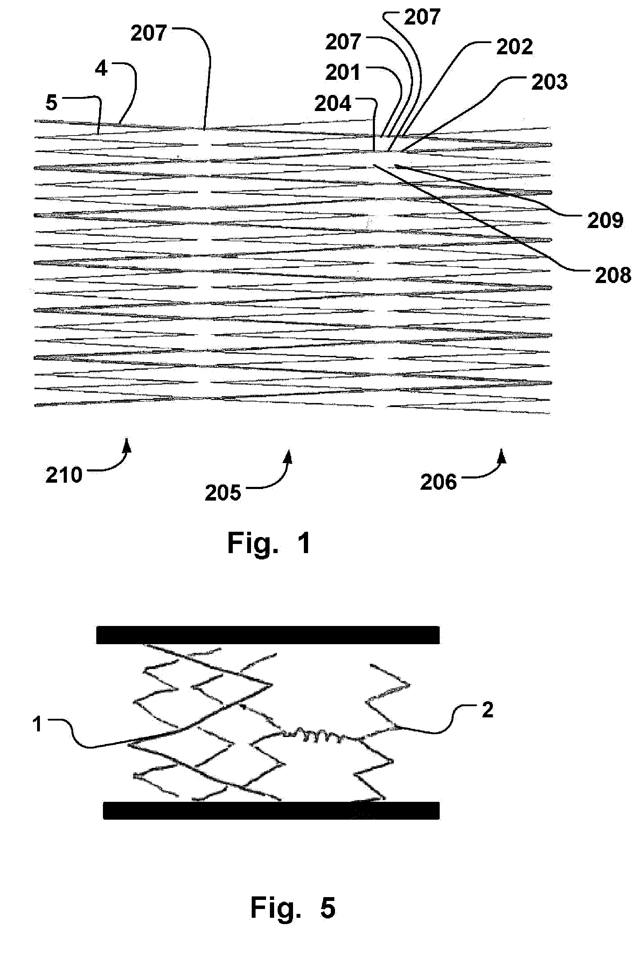

An embodiment of the disclosure according to Fig. 1, which is a lateral view of an endoprothesis 1, will be described below. In Fig. 1 different sections 205, 206, 210 are shown. In Fig. 1 each section 205, 206, 210 comprise struts 5 arranged and connected so as to make up a circumferential zigzag pattern of struts 5. This pattern is called the second circumferential zigzag pattern below. In each of the sections 205, 206, 210, there is such a circumferential zigzag pattern. The struts 5 are of a certain size. This size can be length, diameter or both. Another circumferential zigzag pattern, the one or more first circumferential zigzag patterns, is also present in Fig. 1, namely the zigzag pattern of struts 4 running through all of the sections 205, 206, 210. The struts 4 of this zigzag pattern can be of a different size, such as length, diameter or both, than the other struts 5. However, the struts 4 could also be of a different material than the material used for the other struts 5.

The other struts 5 are preferably smaller than the struts 4, so that the struts 4 allow for a low circumferential force, without sacrificing strength. The other struts 5 will give additional wall coverage as well as an elastic force perpendicular to the surface of the endoprothesis.

The struts 4 comprise sub portions 207, which are connecting the different sections 205, 206, 210 of the support structure. Undesired folding of the endoprothesis is avoided by the use of the differently sized struts.

Preferably the one or more first circumferential zigzag patterns and at least two second circumferential zigzag patterns are overlapping, i.e. integrated in the same cylindrical plane.

Hence, preferably the one or more first circumferential zigzag patterns and at least two second circumferential zigzag patterns are integrated in the same cylindrical plane, and portions of the one or more first circumferential zigzag patterns constitute portions of each of at least two second circumferential zigzag patterns, i.e. some of the struts 5 are sub portions of the struts 4. However, the two different circumferential zigzag patterns could also be joined together with a technique, such as welding or the sub portions 207 could be some kind of connectors, connecting the different sections 205, 206, 210.

Referring again to Fig. 1, a link unit 207 connects the first section 205 with the second section 206 by connecting a first apex 201 of the second circumferential zigzag pattern present in the first section 205 with a first adjacent apex 202 of the second circumferential zigzag pattern in the second section 206. Another link unit 207 connects a second apex 204 of the second circumferential zigzag pattern in the first section 205 with a second adjacent apex 203 of the second circumferential zigzag pattern in the second section 206. The second apex 204 is adjacent to the second adjacent apex 203 and also adjacent to the first apex 201 in another direction.

Furthermore, the first section 205 is connected with the second section 206 with a repeated geometrical tubular pattern. The geometrical tubular pattern comprises a first sub portion 207 of the struts of the first size 4 situated between a first pair of adjacent apexes 201, 202, which comprises the first apex 201 and the first adjacent apex 202. The first sub portion 207 is connecting the first section 205 with the second section 206. The first sub portion 207 is in a radial direction followed by a second sub portion 207 of the struts of the first size 4. The second sub portion is situated between a second pair of adjacent apexes 203, 204, comprising the second apex 204 and the second adjacent apex 203. The second pair 203, 204 is adjacent to the first pair 201, 202, and the second sub portion 207 is connecting the first section 205 with the second section 206. The second sub portion is in the radial direction followed by a third pair of adjacent apexes 208, 209, comprising a third apex 208 of the first section 205, and a third adjacent apex 209 of the second section 206. The third apex 208 is adjacent to the second apex 204 and the third adjacent apex 209 is also adjacent to the second adjacent apex 203. The third pair 208, 209, being adjacent to the second pair 203, 204, is not connecting the first section 205 with the second section 206. Instead, there is an open space between the apexes 208, 209 of the third pair. The geometrical tubular pattern, comprising three apex pairs, is repeated in a radial direction.

In some embodiments an angle between a strut of a first size 4 and a center axis of the tubular support structure is essentially the same as an angle between a strut of a second size 5 and a center axis of the tubular support structure.

In some embodiments the sub portions 207 are link units and the link units 207 are substantially shorter than struts of a second size 5 and/or substantially shorter than the sections 205, 206, 210.

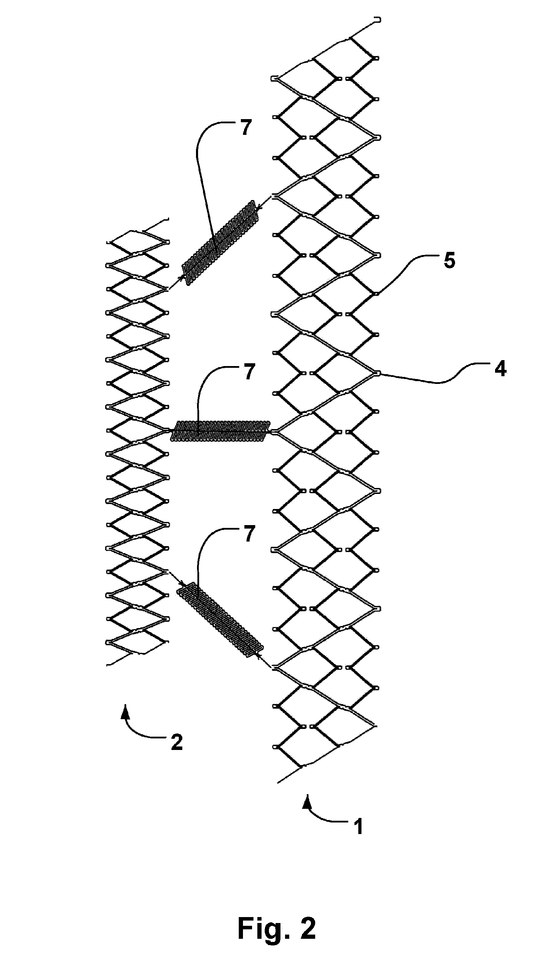

A further embodiment of the disclosure is illustrated in Fig. 2, which is another lateral view of an endoprothesis, in which the structure of the endoprothesis 1 is shown.

Also in Fig. 2 the struts of a first size 4 and the struts of a second size 5 are shown. In Fig. 2, at least one element 2, such as an anchor or fixation part, is attached to the endoprothesis 1 via at least one interconnecting element 7, such as a hinge.

Furthermore, the at least one element 2 comprises at least one distal element and/or at least one proximal element, i.e. there can be elements 2 on either or both sides of the endoprothesis. The element or elements 2 improves the positioning of the endoprothesis.

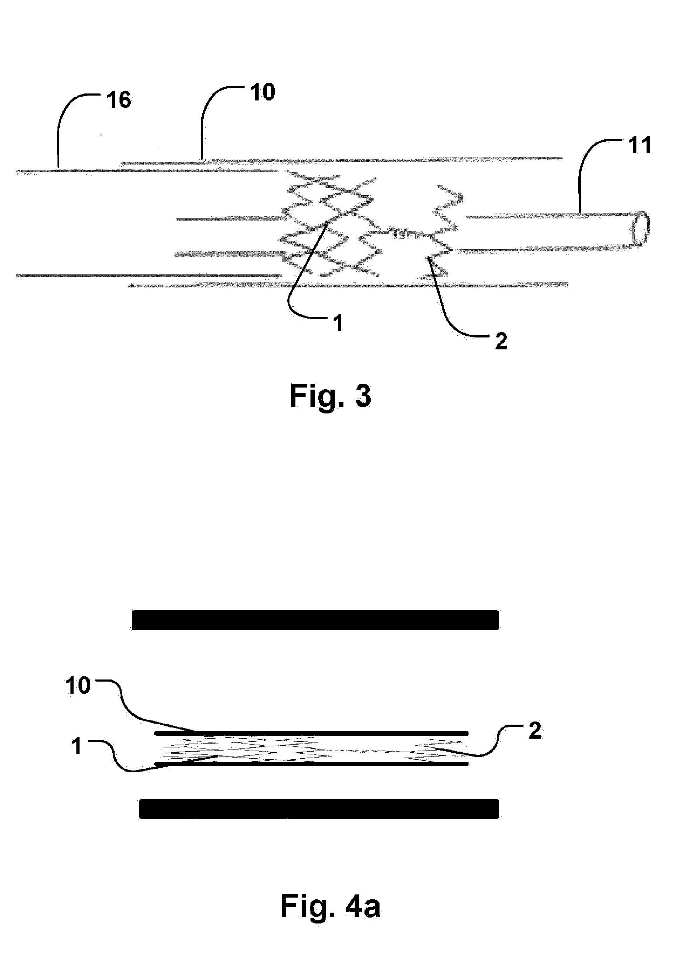

The interconnecting element or elements 7 can be fitted to affix the element 2 with an angle to an outer catheter 10. The outer catheter is shown in figure 3 and it is used for delivery, when released, with the endoprothesis 1 remaining in the catheter. Alternatively, the interconnecting element or elements 7 can be fitted to affix the endoprothesis 1 with an angle to an outer catheter 10 when the endoprothesis 1 is released, with the element 2 remaining in the outer catheter 10.

The interconnecting element or elements 7 are preferable flexible in a longitudinal direction of the interconnecting element or elements 7, e.g. the length is flexible, i.e. the interconnecting elements are stretchable. Thus, the positioning of the endoprothesis is further improved.

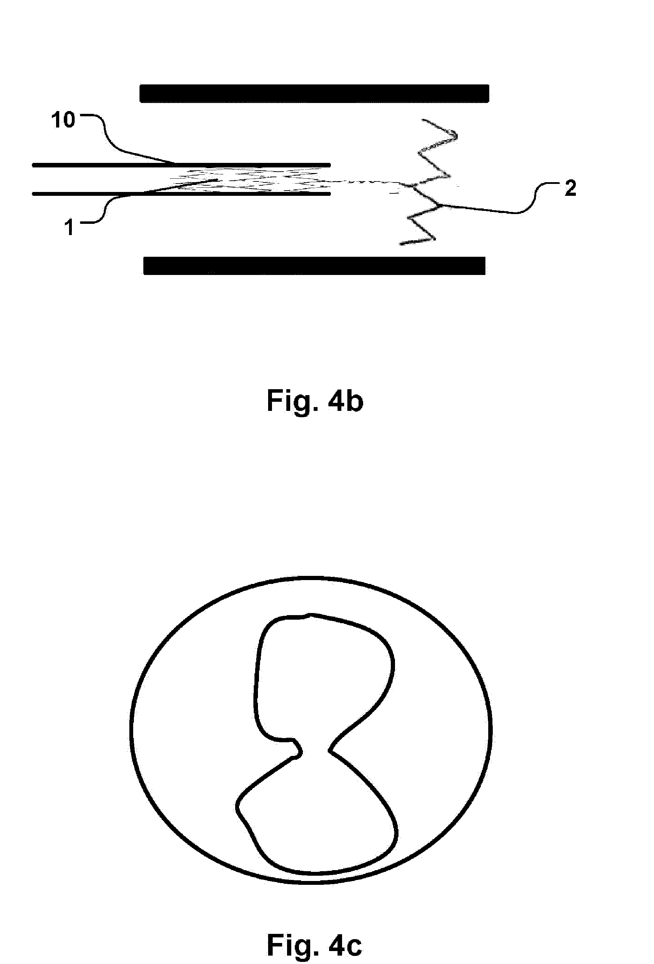

An example of the process of releasing the different parts is further depicted in Figs. 4a, 4b, 4c and 5. Fig. 4a shows the endoprothesis 1 with the element 2 inside the outer catheter. In this figure, the endoprothesis is in its contracted shape for delivery.

Fig. 4b shows a situation where the element 2 has been released and where the actual endoprothesis 1 still remains in the outer catheter 10. However in some applications the endoprothesis 1 may be released before the element 2.

Fig. 4c shows the circumferential shape formed by the element 2, when released. The shape is in this case an eight-shape, since there are only two interconnecting elements 7 between the element 2 and the endoprothesis 1. With three interconnecting elements 7, a trefoil symmetry would have been formed and with four interconnecting elements 7, a four-leaf clover would have been formed. Thus, by the choice of the number of interconnecting elements 7, a desired cross-sectional shape can be formed and undesired cross-sectional shapes can be avoided and the folding issues overcome and thus the flow through the vessel can be further improved. The number of interconnecting elements 7 could be different from the ones given above, i.e. there could be more than 4 interconnecting elements, e.g. 5, 6, 7, 8, 9, 10, 11, 12, 13, 14, 15, 16 etc.

Fig. 5 shows the endoprothesis 1 with the element 2 fully released from the outer catheter 10.

Fig. 3 shows an inner catheter 11 used for delivering a further object. The endoprothesis 1 may also be delivered with a safety wire or a guide wire. Thus a safer delivery mechanism may be provided, the delivery may be simplified and/or the delivery of further objects with the same delivery mechanism may be enabled.

One embodiment of this disclosure is a method of delivering an endoprothesis 1, preferable a self-expanding endoprothesis 1, through a body opening to a target site in a body, which comprises positioning an endoprothesis 1 inside an outer catheter 10. The method comprises insertion of the outer catheter 10 together with the endoprothesis 1 into the body and positioning a distal end of the outer catheter 10 and the endoprothesis 1 at the target site inside the body opening. Moreover, the method comprises deliverance of the endoprothesis 1 to the target site within the body through the outer catheter 10 and removal of the outer catheter 10 from the body.

Optionally in this embodiment, the method may also comprise positioning of an inner catheter 11 or a guide wire inside or in connection with an endoprothesis 1. The method may also comprise positioning of a pushing catheter 16 inside the outer catheter 10 adjacent to the endoprothesis 1, further away from the target site than the endoprothesis 1 prior to the insertion into the body. Furthermore, the method may comprise insertion of the pushing catheter 16 together with the endoprothesis 1 and the outer catheter 10 into the body. Moreover, the method may comprise pushing of the endoprothesis 1 with the pushing catheter 16 until the endoprothesis 1 or the element 2 has been released, so that an element 2 of the endoprothesis 1 is positioned at the target site in its released shape and with a predetermined angle to the outer catheter 10. Thereafter removal of the pushing catheter 16; and further removal of the outer catheter 10, so that another part of the endoprothesis 1, is released and expanded with the predetermined angle, preferably perpendicularly with the outer catheter and in parallel with the element 2, may be performed. The order of releasing the different parts may also be the opposite, so that the endoprothesis 1 is released first and the element 2 thereafter. Thus, the positioning of the endoprothesis may be improved.

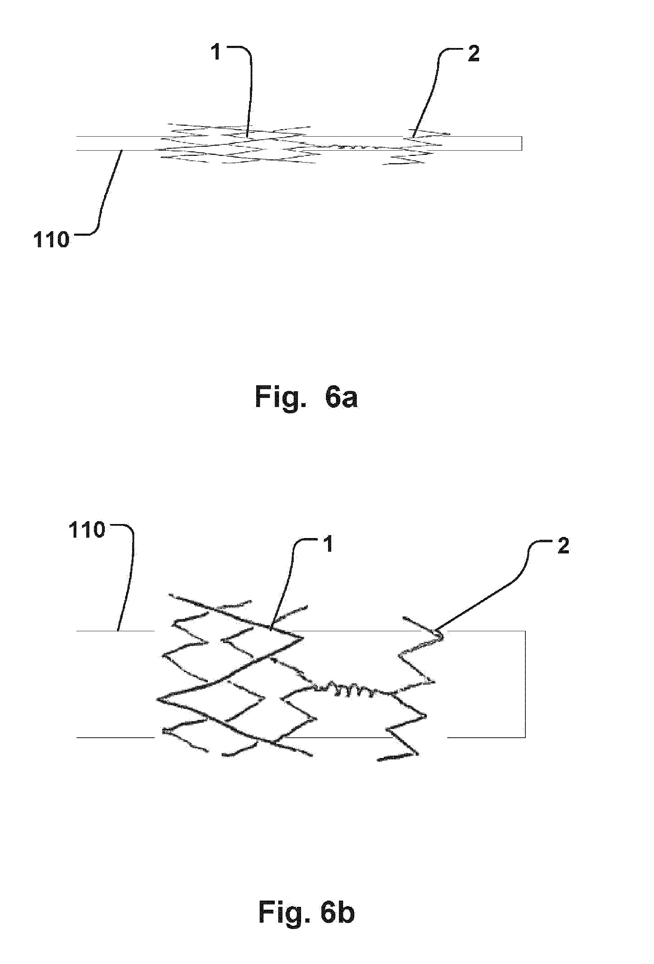

Another embodiment of this disclosure is a method of delivering an endoprothesis 1, preferable an external force expandable endoprothesis 1, through a body opening to a target site in a body. This method comprises: positioning of an endoprothesis 1 outside a delivery element, such as a balloon 110 (shown in Figs. 6a and 6b), which delivery element 110 is short and not substantially longer than the endoprothesis 1 with the element 2. The method further comprises: insertion of the delivery element 110 together with the endoprothesis 1 into the body. Furthermore, the method comprises positioning of the delivery element 110 and the endoprothesis 1 at the target site. Moreover, the method comprises expansion of the delivery element 110, preferably by inflation, thereby expanding the endoprothesis 1 and further expansion of the endoprothesis 1 into its released shape. The method also comprises contraction of the delivery element 110, preferably by deflation; and removing of the delivery element 110. This method aids in avoiding issues with prior art such as long balloon overhang and unnecessary expansion of healthy vessel area.

Yet another embodiment of this disclosure is a method of manufacturing the tubular support structure, which comprises: provision of a solid tube, the solid tube being selected from a group comprising stainless steel, titanium, nickel titanium, i.e. nitinol, tantalum, magnesium, cobalt, chromium, a magnesium alloy or a resorbable polymer. Furthermore, the method optionally comprises grinding of an outer surface of the tube and heat treating annealing the tube. Moreover, the method comprises cutting of the tube to predetermined lengths for formation of the endoprothesis 1. Laser cutting of the outer surface of the solid tube to a predetermined wall surface pattern including the one or more first circumferential zigzag patterns and the at least two second circumferential zigzag patterns in a repeated pattern along the longitudinal axis of the endoprothesis 1 may be performed. Alternatively, the method comprises forming from a first part of the tube, the one or more first circumferential zigzag patterns, and forming from at least a second part of the tube or at least a part from another tube, the at least two second circumferential zigzag patterns and joining the parts together with a technique, such as welding. Moreover, the method comprises sterilization of the endoprothesis 1. The method may comprise crimping of the endoprothesis 1 onto a balloon 110 for direct placement of the endoprothesis 1 if the endoprothesis is an external force expandable endoprothesis 1. Alternatively, if the endoprothesis is a self-expanding endoprothesis 1, the method may comprise putting the endoprothesis 1 into an outer catheter 10. Packaging of the endoprothesis 1 in order to preserve the sterilized condition of the endoprothesis 1 until use is also performed.

Although the tubular structure is described above as having at least two second circumferential zigzag patterns, any feasible number of second circumferential zigzag patterns could be used.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. Furthermore, "connected" or "coupled" as used herein may include wirelessly connected or coupled. As used herein, the term "and/or" includes any and all combinations of one or more of the associated listed items.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The present disclosure has been described above with reference to specific embodiments. However, other embodiments than the above described are equally possible within the scope of the disclosure. Different method steps than those described above, may be provided within the scope of the disclosure. The different features and steps of the disclosure may be combined in other combinations than those described. The scope of the disclosure is only limited by the appended patent claims.

## Claims

1. A tubular support structure for avoiding folding of an endoprothesis (1), such as a stent or a valve, comprising:
one or more first circumferential zigzag patterns;
at least two second circumferential zigzag patterns; and wherein
said first and second circumferential zigzag patterns are overlapping.

2. The tubular support structure of claim 1, wherein
said one or more first circumferential zigzag patterns comprises
struts of a first size (4), such as a length and/or a diameter,
and wherein said at least two second circumferential zigzag patterns
form at least a first and a second section (205, 206) of said tubular support structure, and
wherein each of said second circumferential zigzag patterns comprises
struts of a second size (5), such as a length and/or a diameter, said second size being different, preferably smaller then said first size, and wherein
sub portions (207) of said struts of said first size (4) are connecting said first and second section (205, 206) of said support structure, such that said folding is avoided by said differently sized struts.

3. The tubular support structure of claim 1 or 2, wherein said one or more first circumferential zigzag patterns and said at least two second circumferential zigzag patterns are integrated in the same cylindrical plane, whereby portions of said one or more first circumferential zigzag patterns constitute portions of each of said at least two second circumferential zigzag patterns.

4. The tubular support structure of any of claims 1-3, wherein
an angle between a strut of a first size (4) and a center axis of said tubular support structure is essentially the same as an angle between a strut of a second size (5) and said center axis of said tubular support structure.

5. The tubular support structure of any of claims 2-4, wherein
said one or more first circumferential zigzag patterns and said at least two second circumferential zigzag patterns are cut out from a tube.

6. The tubular support structure of any of claims 2-5, wherein said second size of said struts (5) is length, and wherein said sub portions are link units (207) and wherein said link units (207) are substantially shorter than said struts of said second size (5) and/or substantially shorter than said first and second sections (205, 206).

7. The tubular support structure of claim 6, wherein
said link units (207) connect said first section (205) with said second section (206) by connecting a first apex (201) of said second circumferential zigzag pattern in said first section (205) with a first adjacent apex (202) of said second circumferential zigzag pattern in said second section (206) and
connecting a second apex (204) of said second circumferential zigzag pattern in said first section (205), said second apex (204) being adjacent to said first apex (201), with a second adjacent apex (203) of said second circumferential zigzag pattern in said second section (206).

8. The tubular support structure of claim 7, wherein said first section (205) is connected with said second section (206) with a repeated geometrical tubular pattern, such that:
a first sub portion (207) of said struts of said first size (4) between a first pair of adjacent apexes (201, 202), comprising said first apex (201) and said first adjacent apex (202), is connecting said first section (205) with said second section (206) and is in a radial direction followed by
a second sub portion (207) of said struts of said first size (4) between a second pair of adjacent apexes (203, 204), comprising said second apex (204) and said second adjacent apex (203), said second pair (203, 204) being adjacent to said first pair (201, 202), and which second sub portion (207) is connecting said first section (205) with said second section (206) and is in said radial direction followed by
a third pair of adjacent apexes (208, 209), comprising a third apex (208) of said first section (205), said third apex (208) being adjacent to said second apex (204), and a third adjacent apex (209) of said second section (206), said third adjacent apex (209) being adjacent to said second adjacent apex (203), said third pair (208, 209) being adjacent to said second pair (203, 204) and which third pair of adjacent apexes (208, 209) is not connecting said first section (205) with said second section (206).

9. An endoprothesis (1), such as a stent, comprising the support structure of any of claims 1-8.

10. The endoprothesis of claim 9, wherein a diameter of said endoprothesis (1) is substantially larger than a length of said endoprothesis (1).

11. The endoprothesis of claim 9 or 10, wherein said endoprothesis (1) is delivered with a safety wire, a guide wire and/or outside an inner catheter (11) used for delivering a further object.

12. The endoprothesis of any of claims 9-11, wherein at least one element (2), such as an anchor or fixation part, is attached to the endoprothesis (1) via at least one interconnecting element (7), such as a hinge, and wherein said at least one element (2) comprises at least one distal element and/or at least one proximal element.

13. The endoprothesis of claim 12, wherein said at least one interconnecting element (7) is fitted to affix said element (2) with an angle to an outer catheter (10), used for delivery, when released, with said endoprothesis (1) remaining in said catheter or fitted to affix said endoprothesis (1) with an angle to an outer catheter (10) when said endoprothesis (1) is released, with said element (2) remaining in said outer catheter (10).

14. The endoprothesis of claim 13, wherein said at least one interconnecting element (7) comprises a plurality of interconnecting elements (7) so that the element (2) when released forms a non-round shape, such as an eight-shape, a trefoil symmetry or a four-leaf clover, cross-sectionally.

15. A method of manufacturing the tubular support structure of any of claims 1-8, comprising:
providing a solid tube, said solid tube selected from a group comprising stainless steel, titanium, nickel titanium, tantalum, magnesium, cobalt, chromium, a magnesium alloy or a resorbable polymer;
heat treating annealing said tube;
cutting said tube to predetermined lengths for formation of said endoprothesis (1);
laser cutting said outer surface of said tube to a predetermined wall surface pattern including said one or more first circumferential zigzag patterns and said at least two second circumferential zigzag patterns in a repeated pattern along the longitudinal axis of said endoprothesis (1) or
forming from a first part of said tube, said one or more first circumferential zigzag patterns, and forming from at least a second part of said solid tube or at least a part from another solid tube, said at least two second circumferential zigzag patterns and joining said parts together with a technique, such as welding;
sterilizing said endoprothesis (1);
crimping said endoprothesis (1) onto a balloon (110) for direct placement of said endoprothesis (1) if said endoprothesis is an external force expandable endoprothesis (1) or putting said endoprothesis (1) into an outer catheter (10) if said endoprothesis is a self-expanding endoprothesis (1); and
packaging said endoprothesis (1) to preserve the sterilized condition of said endoprothesis (1) until use.
